# EUROPEAN PATENT APPLICATION

(11) **EP 0 747 477 A1**
(43) Date of publication of application: **11.12.1996**
(21) Application number: 94906413.3
(22) Date of filing: 28.01.1994
(51) Int. Cl.: C12N 9/06, C12R 1/885

(54) **STRAIN OF TRICHODERMA HARZIEANUM RIFAI, PROCESS FOR OBTAINING L-LYSINE-ALPHA-OXIDASE AS AN INHIBITOR OF VIRAL AND BACTERIAL ACTIVITY, IMMUNOMODULATOR AND SKIN HEALING AGENT**

(71) Applicant: Smirnova, Irina Pavlovna, Moscow 123423 (RU); Alexeev, Sergei Borisovich, Moscow 119049 (RU); Berezov, Temirbolat Tembolatovich, Moscow 117437 (RU); Andzhaparidze, Otar Georgievich, Moscow, 103051 (RU); Zgursky, Alexandr Alexandrovich, Moscow 12362 (RU); Stepanova, Lidia Griorievna, Moscow 103055 (RU); Zverev, Vitaly Vasilievich, Moscow, 123060 (RU); Goltzov, Viktor Alexandrovich, Moscow, 115477 (RU); Diorditsa, Sergei Viktorovich, Moscow 123585 (RU); Vesa, Vitautas Simonovich, Vinjus, 232015 (LT)
(72) Inventor: Smirnova, Irina Pavlovna, Moscow 123423 (RU); Alexeev, Sergei Borisovich, Moscow 119049 (RU); Berezov, Temirbolat Tembolatovich, Moscow 117437 (RU); Andzhaparidze, Otar Georgievich, Moscow, 103051 (RU); Zgursky, Alexandr Alexandrovich, Moscow 12362 (RU); Stepanova, Lidia Griorievna, Moscow 103055 (RU); Zverev, Vitaly Vasilievich, Moscow, 123060 (RU); Goltzov, Viktor Alexandrovich, Moscow, 115477 (RU); Diorditsa, Sergei Viktorovich, Moscow 123585 (RU); Vesa, Vitautas Simonovich, Vinjus, 232015 (LT)
(74) Representative: Patentanwälte Zellentin & Partner
(86) International application number: RU9400012
(87) International publication number: WO9520650

(57) **Abstract**

The present invention relates to a new strain *Trichoderma harzianum Rifai* which produces a ferment of L-lysine-α-oxidase with a broad spectrum of biological activity. The invention also relates to the use of L-lysine-α-oxidase as an antiviral, antibacterial, immunomodulating and healing agent. Also disclosed is a process for obtaining L-lysine-α-oxidase involving the culture of *Trichoderma sp*..

## Description

The present invention pertains to the field of biotechnology and medicine, namely to a new strain of Trichoderma harzianum Rifai - the producer of L-lysine-α-oxidase, a process for preparing L-lysine-α-oxidase using the new strain. L-lysine-α-oxidase produced according to the invention possesses a wide spectrum of biological activity. The invention also pertains to the application of L-lysine-α-oxidase produced by the Trichoderma harzianum Rifai strain in different fields of medicine, in particular, as an inhibitor of viral and bacterial activity, as an immunomodulator, as well as a stimulator of healing of skin lesions.

The interest in oxidases of L-amino acids catalysing the reaction of oxidative deamination of L-amine acids with the formation of α-ketoacid arose in late 70ies-early 80es after publication of the studies by Soda K. and his colleagues on the isolation of the enzyme from the Trichoderma viride (Y-244-2) fungus and demonstration of the prospects of the application thereof as an antitumor agent (N.Kusakabe, K.Kodama, A.Kuninaka, H.Yoshina, K .Soda- Agricultural Biol.Chem., 1980, 44:2, 387-392) inhibiting the growth of leukemic cell lines in vivo and in vitro.

The antitumor properties of L-lysine-α-oxidase isolated from Trichoderma sp. were studied by Berezov and coworkers (Berezov, T.T., Khaduev, S.Kh., Lukashova, E.V., Smirnova, I.P. - The mechanisms of cytostatic effect of L-lysine-α-oxidase from Trichoderma sp., 14th Intern. Cancer Congr., August 21-27, Budapest, 1986, v.3, p.933) and other groups of investigators (I.C. USSR No. 1660387 and Experim. Oncology, 1985, v.7, No.6, p.42-44).

A new strain of Trichoderma harzianum Rifai isolated by the authors (deposited on 12.01.82 in the collection of industrial microorganisms VNII Genetica under the number F-180) has a high activity (3.3-8.0 conditional units of activity) which is 2.0-2.5-fold exceeds that of the known strain (Soda et al., 1.2-3.0 conditional units of activity).

There are known processes for preparing L-lysine-α-oxidase including cultivation of Trichoderma sp.

The method of shallow cultivation of Trichoderma harzianum Rifai is described in I.C. USSR No.1044043, 1982.

The method is as follows: the fungus is grown in a flask of 500 ml capacity in a medium containing NaNO₃ 11.4%-14 ml, wheat bran, distilled water 10 ml. The cultivation goes on at 28°C for 14 days. To the resulting fungus culture 200 ml of water are added, and the enzyme is extracted for 2 hours at room temperature. The extract of L-lysine-α-oxidase is obtained by pressing out the culture grown on the bran through a cloth followed by centrifugation.

There is another shallow method of producing L-lysine-α-oxidase (I.C.USSR No.1212046, cl. C12 N 9/52, 1985) which stipulates cultivation of Trichoderma sp.fungus, a producer of L-lysine-α-oxidase, and a multi-stage process of the enzyme extract obtaining. The method is as follows; the fungus is grown for 8 days at 28°C in a nutrient medium with wheat bran (10%), ammonium sulphate (0.6-1.3 % by weight) and water - 10 ml. To the culture grown in this way 100 ml of water are added under sterile conditions, extracted for 2 hours, and the resulting extract is pored into a flask. The remaining culture is grown for 3-4 days in a thermostat at 28°C after which the second washing of the culture is used.

The known methods, however, have a number of shortcomings, among them low activity of the resulting enzyme and the long duration of the process for obtaining thereof.

The claimed process yields the enzyme with a higher specific activity (up to 1.2 U/mg of protein) within a shorter period of the producer cultivation owing to the submerged cultivation and a certain ratio of the components of the fermentation medium and conditions of cultivation.

L-lysine-α-oxidase produced by the F-180 strain possesses, alongside with the antitumor activity, unexpected biological properties; the capacity to inhibit viral and bacterial activity of gram-positive bacteria, the effect of immunomodulation, and the capacity to stimulate healing of skin lesions.

Antiherpes preparations are known which are acyclonucleosides of the type of acyclovir and flavanoids, among which luteoline is active against herpes simplex virus type 1 (HSV-1) (Wleklik et al., Acta virol., 1988, p.32).

At a concentration of these preparations of 50-100 µg/ml the inhibitory effect assessed by a reduction of CPO₅₀ (a dose producing the cytopathic effect in 50% of the cells) on the cells cultivated in vitro was 3.5-4 x 100. These preparation used for a long time have a toxic effect and large doses of the preparations are required for the achievement of a positive result. Besides, the said preparations are not effective for all types of herpes simplex virus.

Lysine-oxidase produced by the strain according to the invention is an effective preparation inhibiting HSV reproduction, possessing a higher specificity and a lower toxicity than the preparations of the known prior art.

A limited range of preparations including azizothymidine is used at present as an inhibitor of human immunodeficiency virus (HIV) (M.Mitsuya, S.Broder - strategies for antivirus therapy in AIDS.- Nature, 1987, v.325, No. 6107, p.773-778). All of the known preparations, however, are highly toxic.

L-lysine-α-oxidase according to the invention has a low toxicity and is highly effective.

Proteclytic enzymes are known to be used for treatment of skin lesions (T.Ya. Dubrovina et al.- "Mechanisms of Pathogenesis of Viral Infections"_{,} 1991, p.3-7, AMS, Leningrad).

The wound-healing properties of aminooxidases have been unknown until the present invention. The claimed L-lysine-α-oxidase manifests highly specific wound-healing effect.

A number of preparations are used as immunomodulators, among them interferons, interleukins (T.Ya. Dubrovina et al.-"Mechanisms..."p.3-7 AMS, Leningrad). The authors of the invention have first discovered the immunomodulating properties of L-amino acids oxidases.

### 1. The strain. Morphological and biochemical properties.

The claimed strain Trichoderma harzianum Rifai F-180 is characterized by the following morphological and physiological-biochemical properties.

### Morphological properties

The colonies grow fast in the wort-agar medium. The mycelium is colourless, septate, prostrate. At 4-5 days of growth there appear mats with pulvinate conidiophores, initially white, later of yellowish- or dark green colour. Phialides are lageniform (9-12, µ), located as verticils of 3 and more. On each phialide conidia are formed glued into a head. The conidia are rounded, smooth, small (3-5 µ), pale green in the passing light and dark in mass. In the agar-containing Chapek medium the colonies grow fast but poorly form the spores.

### Biochemical properties

The strain grows well in Capek medium, however the wort-agar medium is preferable for the maintenance of the culture. The culture assimilates well different forms of nitrogen, both nitrate (NaNO₃, KNO₃) and ammonated (NH₄)₂SO₄. The latter form is assimilated much faster which leads to the acidification of the medium favourable for the better growth of the culture.

Among organic compounds, peptone is the best source of nitrogen.

The culture grows well in the medium containing asparagine and glutamic acid.

Among carbon sources it assimilates well glucose, xylose, sucrose, lactose, galactose, maltose.

Intensive growth is observed in media with sugars. Poorly assimilated are methyl alcohol, ethyl alcohol, dulcitol.

The culture may utilize complex sources of carbon. It growth well on wheat bran. The enzyme L-lysine-α-oxidase is induced only when bran is used as the source of carbon.

The use of synthetic media with maltose, glucose, alcohol results in a decrease of L-lysine-α-oxidase activity.

L-lysine-α-oxidase is formed during the first stages of the culture growth, later, L-phenylalanine-α-oxidase activity and L-methionine-α-oxidase activity are manifested in a ratio of 100: 8.6: 0.7, respectively.

The strain is highly specific. However, when the conditions of cultivation are changed (more complicated composition of the medium), the oxidase activity may be manifested with the amino acids L-arginine, L-histidine, L-tyrosine, L-leucine.

The culture grows very well in wort-agar, and this medium may be used for the culture maintenance. A lower intensity of growth and formation of spore-bearing are observed in potato-dextrose agar. Capek medium may also be used for the maintenance of the culture.

The culture does not liquefy agar or gelatin, does not peptonize milk, does not decompose starch and represents an aerobic fungus. The optimal temperature for growth is 28°-29°C but it can also grow in the range of 24°-30°C. The optimal pH for the culture growth is 5.6 however, the growth may also be observed in the pH range of 1.5 to 9. The culture produces a large number of spores in the wort-agar medium. The culture is stored in a medium with wheat bran under the above-described conditions for a period up to 1 year.

### II. The process for preparing L-lysine-α-oxidase

The claimed process provides for submerged cultivation of the strain Trichoderma harzianum Rifai F-180 in a fermentative medium at the following ratio of components (in % by weight): wheat bran 4-6, the source of inorganic nitrogen 0.7-0.9, water - the rest, pH of the medium is 5.5-6, and cultivation is carried out under conditions of aeration at a rate of 100-130 rpm.

In the preferable variant of the process embodiment, to obtain the culture fluid the seed material (slopes with the culture) is grown in the wort-agar medium for 14 days in a thermostate at 28°C. In order to obtain the inoculum for the submerged cultivation, a medium with wheat bran of the following composition is used: wheat bran 20 g, NaNO₃ 11.4%, solution 1.4 ml, H₂O 10 ml.

The inoculum is grown in a thermostate at 28°C for 12-14 days and is used for seeding on a fermentative medium. Trichoderma fermentation is varried out in flasks of 250 ml capacity on a thermostatic shaker of the type 357 (Poland) at 28°C for 5 days, with the amplitude No.6, 120 rpm.

The fermentative medium has the following composition: wheat bran 5 g, NaNO₃ 0.9 g, H₂O up to 100 g. The initial pH of the medium is 5.5-6. The amount of the incculum grown on the solid medium with wheat bran is 0.5-1 g of the total amount. Trichoderma harzianum Rifai F-180 is grown by the submerged method for 4-5 days at 28°C. The resulting supernatant is used as the source of the enzyme.

The activity of L-aminoacide oxidases is determined spectrophotometrically by the amount H₂O₂ formed in the process of fermentative reaction which is determined spectrophotometrically using orthodianisine micromethod. The essence of the method consists in the interaction of all H₂O₂ formed in the reaction with O-dianizidine hydrochloride. The incubation medium contained 20 µg peroxidase, 250 µg O-dianizidine hydrochloride and 0.1-0.5 mg protein in 1 ml of the final volume. The optic density of stained solutions of the experimental and control (without the substrate) specimens was measured in SF-16 spectrophotometer.

The unit of the enzyme activity is considered to be the amount of the enzyme catalysing the production of 1 nmol H₂O₂ per minute under the standard conditions per 1 ml of fluid or 1 mg of protein.

The influence of the wheat bran amount on the L-lysine-α-oxidase activity of the fungus is shown in Table 1.

The effect of the source of nitrogen on the L-lysine-α-oxidase activity of the fungus is shown in Table 2.

With the decrease of the amount of NaNO₃ the synthesis of the enzyme decreases, and its higher content facilitates alkalinization of the medium which inhibits the growth of the fungus culture.

**Table 1**

| The amount of wheat bran, % by weight | L-lysine-α-oxidase activity, U/mg of protein |
|---|---|
| 2.5 | 0.6 |
| 4 | 1.0 |
| 5 | 1.2 |
| 6 | 1.0 |
| 7 | 0.9 |
| 10 | 0.8 |

**Table 2**

| Nitrogen source (NaNO₃), % by weight | L-lysine-α-oxidase activity, U/mg of protein |
|---|---|
| 0.50 | 0.6 |
| 0.7 | 1.0 |
| 0.9 | 1.2 |
| 1.25 | 0.7 |
| 1.50 | 0.6 |

The effect of unitial pH of the medium on biosynthesis of L-lysine-α-oxidase (the 5th day of growth) is shown in Table 3.

**Table 3**

| Initial pH of the medium | Activity, U/mg |
|---|---|
| 5.0 | 0.4 |
| 5.4 | 1.0 |
| 5.6 | 1.2 |
| 6.0 | 0.9 |
| 6.2 | 0.5 |

The effect of different sources of nitrogen on biosynthesis of L-lesine-α-oxidase is shown in Table 4.

**Table 4**

| Nitrogen source | Activity, U/mg of protein |
|---|---|
| NaNO₃ | 1.0 |
| (NH₄)₂SO₄ | 0.8 |

As will be seen in Table 4, different forms of inorganic nitrogen are used for biosynthesis of the enzyme by the submerged cultivation.

The effect of aeration on the L-lysine-α-oxidase (LO) activity is shown in Table 5.

**Table 5**

| Day of growth | Activity, U/mg LO |
|---|---|
| at 100 rpm | |
| 2 | 0.43 |
| 4 | 0.56 |
| 6 | 0.52 |

| at 120 rpm | |
|---|---|
| 2 | 1.10 |
| 4 | 1.18 |
| 6 | 0.78 |

| at 130 rpm | |
|---|---|
| 2 | 0.7 |
| 4 | 0.9 |
| 6 | 0.43 |

At the end of fermentation, (NH₄)₂SO₄ was added to the culture fluid up to 20% saturation, and incubation continued for 3-4 hours. Then the culture fluid was centrifuged at 3000-4000 rpm and the supernatant (about 90 litres) was subjected to further treatment. The LO content was about 4-6 units/mg of supernatant.

Preparation of highly purified L-lysine-α-oxidase enzyme: to the supernatant obtained above dry (NH₄)₂SO₄ was added to 50% saturation and the material was incubated for 3-4 hours. Centrifugation was 3000-4000 rpm for 30 min. and the supernatant was discarded. The pellet was washed twice with a solution of (NH₄)₂SO₄ (40% saturation) and then dissolved in a buffer containing 20 mM tris-HCL, pH 7.2-7.6. The volume of the buffer was 1/50 - 1 /100 of the volume of the initial culture fluid.

To the resulting solution (1-2 1 volume), diatomaceous earth (bentonite) was added in the amount of 40-50 g/l and mixing went on for 1-1.5 hours. The mixture was filtered through Büchner funnel with a fine glass filter (20-40 µm) under weak vacuum.

The filtrate was loaded on a DEAE-cellulose column of 500-600 ml volume balanced with 10 mM tris-HCl buffer, pH 7.2-7.6 (the volume of the balancing buffer about 5-6 1). The rate of the buffer flow through the column was about 150-200 ml/hour. After the loading of the material the column was washed with a buffer 10 mM Tris-HCl, pH 7.2-7.6, 0.2 M NaCl. The control consisted in measuring of the optic density at 280 nm.

The enzyme was eluted with a buffer 10 mM Tris-HCl, pH 7.2-7.6, 0.5 n NaCl, and fractions were collected which contained the LO activity. As a result, 900 ml of the enzyme was obtained with the specific activity of 150 µU /mg protein. The yield by the activity was 70-75%.

The enzyme L-lysine-α-oxidase prepared by the claimed process possesses the following properties:
- the substrate specificity: subjects L-lysine aminoacid to irreversible oxidative deamination;
- high degree of substrate specificity towards lysine; the value of Michaelis constant (Km) is 1.4+0.1 x 0.01 mM (the substrate - L-lysine),
- the pH optimum of the catalytic activity is 7.4;
- the isoelectric point is 4.25;
- according to the results of gel filtration on sephandex G-200 and gel electrophoresis, the molecular weight is 114+39 kD;
- the enzyme molecule consists of two identical subunits with molecular weight of 60 kD each;
- the enzyme retains 75% and 68% of its activity at 60°C and pH 7.4 after 2 hours of incubation in human blood plasma and Tris-HCl buffer, respectively.

The enzyme is inhibited by 4-azo-D,L-leucine and 6-diazo-5-oxinorleucine.
- The enzyme can be stored frozen or lyophilized for one year without any loss of the enzymatic activity.

### III. Therapeutic application

The product prepared according to the claimed process is useful for medical application. Different forms of the preparation containing L-lysine-α-oxidase according to the invention may be applied for different purposes. In skin lesions, including those caused by herpes simplex virus, solutions, ointments, gels containing L-lysine-α-oxidase in an amount effective for healing of such lesions are used. Any compounds usable in pharmaceutical industry may be used as solvents, excipients, additions. When L-lysine-α-oxidase is used as an immunopotentiating, antibacterial and anti-AIDS substance as well as for treatment of mixed infections, most frequently used are injections of solutions containing L-lysine-α-oxidase in an amount sufficient for the achievement of the therapeutic effect.

### IV. Study of L-lysine-α-oxidase activity in vitro

The cytotoxic effect of lysine-α-oxidase as well as the inhibiting effect of lysine-α-oxidase on the expression of the antigens of herpes simplex virus type I and virus reproduction was studied in green monkey kidney cell line Vero sensitive to the virus. The data on the cytotoxic effect of lysine oxidase on Vero cells are presented in Table 6. A moderate toxicity was determined as that producing the death of 7% to 10% of the cells, toxicity as producing the death of more than 10% of the cells. In control cultures the portion of dead cells did not exceed 5%.

**Table 6**

| The cytotoxic effect of various concentrations of lysine oxidase on continuous cell line Vero | | | | | | |
|---|---|---|---|---|---|---|
| Preparation | Lysine oxidase concentration in U/ml | | | | | |
| | 2.55 | 0.1 | 0.01 | 0.001 | 0.0001 | 0.00001 |
| Lysine oxidase | Toxic | moderately toxic | mod. toxic | not toxic | not toxic | not toxic |

In the experiments investigating the effect of L-lysine-α-oxidase on herpes simples virus reproduction Vero cells were cultivated in vitro according to the standard method (same as in the experiments investigating the cytotoxic effect) in medium RPMI-1640 containing 100 units/ml of ampicilline and 10% of bovine serum. The cells were grown in standard glass tubes at 37°C. The seed dose was 500,000 cells in 1 ml of the culture medium. After a confluent cell monolayer had been formed (control under the light microscope) the cells were inoculated with HSV-1 using doses of 1 and 0.1 PFU/cell (plaque-forming unit). After 30 min of incubation at 37°C the virus-containing fluid was removed, the cells were washed three times with the culture medium and then the culture fluid containing lysine oxidase in different concentrations was added. The inhibition of virus reproduction in Vero cells was determined by a decline of the cytopathic effect of the virus on the cells. The results are presented in Table 7.

**Table 7**

| Inhibition of HSV-1 reproduction by lysine oxidase | | | | | | | |
|---|---|---|---|---|---|---|---|
| Lysine oxidase U/ml | Virus dilution 1,10 CPD₅₀ | | | | | | |
| | 0.1 | 0.01 | 0.001 | 0.0001 | 0.00001 | 0.000001 | 0.0000001 |
| 0.0001 | + | + | + | + | - | - | - |
| 0.001 | + | + | + | - | - | - | - |
| 0.01 | + | + | - | - | - | - | - |
| Control | + | + | + | + | + | + | + |

As will be seen from the above results, a concentration of lysine oxidase 0.01 U/ml (0.3 µg/ml) produced approximately 100,000-fold decrease of the cytopathic effect of HSV-1 on the cells cultivated in vitro.

The inhibition by L-lysine-α-oxidase of HSV-1 antigens expression was determined by immunoblotting method 24 hours after infection of Vero cell cultures. The multiplicity of infection was 1 PFU/cell. One control was the presence of HSV-1 antigens in the infected Vero cells, the other control - uninfected cells. The lysine oxidase concentrations tested were 0.7 ng/ml, 0,07 µg/ml, 0.7 µg/ml.

The culture fluid was removed from the tubes in 24 hours and the cells were taken off the glass in a lysing buffer of the following composition: 8% sodium dodecyl sulphate, 30% glyserine, 5% 2-mercaptoethanol in 1.25 M tris-HCl buffer, pH 6.8. The lysates were heated at 100°C for 1 min after which the proteins present in the lysates were separated by electrophoresis in 0.5 mm block of 12% polyacryl amide gel in the presence of 0.1% SDS according to Laemmly method. Electrophoresis was carried out for 18 hours at a voltage of 50v.

The electric transfer of proteins on nitrocellulose membranes with a pore diameter of 0.45 µm was done at the current power of 500 mA for 1.5 hours. For prevention of non-specific adsorption, the nitrocellulose membranes were blocked with 0.55% Twin-20 in phosphate-buffered saline (PBS-T) for 1 hour, then polyclonal immunoglobulins against HSV-1 diluted 1:200 in PBS-T were added and incubated for 18 hours. The unbound immunoglobulins were removed by 3 washings with PBS-T, incubated with a peroxidase- antispecies immunoglobulins conjugate for 1.5 hours, washed with PBS-T and stained with diaminobenzidine or chloronaphthole according to the standard method.

The results of immunoblotting demonstrated complete inhibition of viral antigen expression (envelope proteins gB, gD and gp80).

### V. Investigation of L-lysine-α-oxidase effect on the development of herpetic disease caused by infection of eyes and skins of rabbits with HSV-1

The study was carried out in Chinchilla rabbits weighing 1000-1100 g in which corneal lesions of both eyes and skin lesions in the shoulder blades infected with HSV-1 were examined. The strain L₂ of HSV-1 obtained from the museum of D.I. Ivanovsky Institute of Virology of the USSR AMS was used for the experiments; it was inoculated in a dose of 10⁵ tissue cytopathic units (TCD₅₀) which constituted 50% of the lethal dose.

The efficacy of the preparation* in herpes disease was assessed by the following parameters:
- daily examinations of the macroscopic picture of the eye and skin diseases;
- immunological examinations of the materials collected at the foci of lesions at 1, 2, 3, 5, and 7 days after infection;
- morphological findings (corneal and skin preparations) at 7 days after infection.

* Trade name "Heralex"

The schedules of treatment of the animals with different medicinal forms of the preparation are presented in Table 8. The treatment of herpetic infection in the area of skin lesions and eye cornea in rabbits was started immediately after infection with HSV-1. The preparation was used in the form of solution and gel. The therapeutic effect could be seen already within 3-4 days postinfection .

In the control group of untreated animals the observations macroscopically revealed edema, lacrimation, marked injection of the blood vessels of the pericorneal and cojunctival zones. In histological preparations the corneal epithelium was absent in quito long areas, throughout the thickness of the layer proper marked edema and cellular, predominantly leukocyte, infiltration were observed.

**Table 8**

| Schedules of rabbits' herpetic conjunctivitis and skin lesions treatment by "Heralex" | | | |
|---|---|---|---|
| Time of administration of the preparation | Site of lesion caused by HSV-1 | Dose of the preparation | Medicinal form |
| Immediately postinfection | eye wound | 0.42 µg/ml | solution |
| | skin wound | | |
| Immediately postinfection | eye wound | 0.42 µg/ml | gel |
| | skin wound | | |
| 24 hours postinfection | eye wound | 4.2 µg/ml | gel |
| | skin wound | | |
| 24 hours postinfection | eye wound | 70.0 µg/ml | gel |

In the group of animals given the preparation in the form of eye drops the structures of the eye cornea recovered partially, in the ulcerative zone a slight thickening of the epithelial layer was observed, the anterior marginal membrane could not be determined definitely and was absent in some sites; at the remaining length the thickness of the corneal epithelium was normal, the cellular infiltration in the layer proper was weak and represented by lymphocytes and eosinophile. The described picture attested to residual inflammatory changes and a weak hyperplastic process typical of early stages of the disease.

In the group of animals treated with the gel form of the preparation the macroscopic and histological findings indicated recovery with complete restoration of the eye cornea; the epithelial layer had limited and occasional sites of thickening, the layer proper and the inner epithelial membrane recovered without changes.

The results of macroscopic and morphological studies mainly coincide with the results of immunological analysis of secretions from eyes of untreated and treated animals. In viral infection of the eyes the preparation inhibited the synthesis of viral proteins, the gel form thereof being more effective than a solution.

The preparation also exerted a positive therapeutic effect on herpetic skin lesions in rabbits. In the control group of the animals the zone of herpetic lesion macroscopically was presented by a crust with an area of hyperpigmentation around it. The epidermis was absent macroscopically, a layer of epithelial cells crawled accross from the edgest of the lesion. The bottom of the lesion was covered with necrotized masses: subepidermally, marked inflammatory infiltration was observed (polymorphonuclear leukocytes, macrophages); on the edge of the lesion, insignificant infiltration with cells of the basal layer of the epidermis and hyperkeratinization were found. These changes in the zone of lesions indicate the persisting acutenes of the inflammatory process both in superficial and deeper layers of the derma.

Administration of the preparation in the form of a solution immediately after infection accelerates the process of healing and formation of the young granulating tissue in the zone of lesion. It may be seen in histological sections that normal epithelization of the skin occurred; the inflammatory infiltration of the superficial layers of the derma was quite limited and represented mainly by accumulations of lymphocytes and fibroblasts.

The treatment of the infected skin areas of rabbits with the preparation in the form of gel was most effective. Macroscopically in the zone of the herpetic lesion the skin was smooth, slightly pigmented, practically of normal structure. Histological sections revealed complete restoration of the normal skin architectonics with the exception of limited areas of epidermis thinning and signs of hyperkeratosis. The method of immunoblotting demonstrated significant differences in HSV-1 proteins expression which, on the whole, confirmed the results of morphological studies. In herpetic lesions of the skin, significant expression of viral proteins was observed throughout the period of the disease, whereas during the treatment of the lesions with the preparation both in the solution and the gel forms the expression of HSV-1 proteins was less marked than in the controls.

The above-presented results of the treatment of herpetic infection with L-lysine-α-oxidase proved the principal possibility of using thereof as an antiviral preparations which is highly effective in the early stages of the disease especially in the medical form of a gel. However, the human patients contracting herpes infection, as a rule, apply for medical help in 2-3 days postinfection, at the time of development of herpetic eruptions. Consequently, the schedule of treatment in which the application of medicinal antiviral preparations begins at 2-3 days postinfection seems to be the closest to the life situations.

In this connection, the efficacy of the preparation in gel was investigated on a model of herpetic conjunctivitis and herpetic skin lesions in rabbits infected with HSV-1. The treatment was started 24 hours after infection. The results of macroscopic and histological studies as well as the results of immunological analysis indicated that the preparation in doses of 1.4-3 µg/ml exerted a significant therapeutic effect, however, some residual changes may persist.

The use of the preparation in higher doses one day after infection demonstrated its high effectiveness in the treatment of both herpetic conjunctivitis and herpetic skin infection (complete restoration of the structure of skin or cornea).

The results of immunological analysis coincided with the pattern of morphological lesions revealed in treatment of herpetic eye and skin lesions. These results are presented in Table 9 in which it can be seen that the inhibition of virus-specific proteins in HSV-affected areas of the skin was slightly weaker than that observed in herpetic conjunctivitis, but by the 6th day there was no expression of viral antigens in acrappings from the eyes or the skin of the laboratory animals. Besides, no toxix or allergic effects of the gel form upon rubbing thereof into the skin and eyes of normal animals have been registrated.

**Table 9**

| The effect of L-lysine-α-oxidase on HSV-1 antigens expression determined by immunoblotting from the lesions | | | | | |
|---|---|---|---|---|---|
| Day | Control (normal animals) | Herpetic conjunctivitis | Herpetic skin lesions | Herpetic conjunctivitis + preparation (70 µg/ml) | Herpetic skin lesions + preparation (70 µg/ml) |
| 2 | - | + | + | + | + |
| 4 | - | + | + | + | + |
| 6 | - | + | + | - | - |

### VI. The effect of L-lysine-α-oxidase on the development of herpes simplex virus infection of guinea pig genitals

In this study, herpes simplex virus type 2 (HSV-2), the strain MS obtained from the USA National Collection of viruses (Rockville, Maryland, USA) is used. The virus was maintained by passages in susceptible Vero cell culture in RPMl-1640 medium containing 500 U/ml benzylpenicillin.

The virus isolated from the lesions was titrated in vero cell cultures by the standard method and the results were expressed in TCD₅₀.

Male guinea pigs weighing 300 g were used in the experiments. Before inoculation the animals received intramuscular injections of 300 mg/ml ketalar for anesthesia. 0.1 ml of the virus suspension containing a dose of 10³ PFU/ml (LD₁₀₀) was applied to the preliminarily scarfied muccsa of the sex organ of each animal. The clinical manifestations of the acute herpetic infection of the genitals were assessed in guinea pigs by a 5-score system.

Several groups of animals were used in the experiments: the 1st group - control, the animals received no treatment; the 2nd group - the animals received once daily combined treatment with L-lysine-α-oxidase preparations by application of 0.5 ml gel (a concentration of 70 µg/ml) on the lesions and injections of aqueous solution of the preparation in a volume of 0.25 ml (concentration of the preparation 100 µg/ml);
the 3rd group - the animals received daily a single application of L-lysine-α-oxidase preparations of 0.5 ml gel in which the preparation concentration was 70 µg/ml on the site of the sex organ mucosa infected with herpes virus.

In the control group of animals, typical local manifestations of genital herpes developed within 24-48 hours postinfection. Initially, single vesicular eruptions appeared on the mucosa of the sex organ. With the progress of the disease the number of vesicles increased, confluent foci with hemorrhages appeared. By the 4th-5th day the symptoms of generalization of the infection developed such as fever, flabbiness, paresis of the pelvic organs, and paralysis of the hind limbs. With the increasing sings of meningo-encephalitis by the 7th-8th day the lethal outcome occurred.

Both topical and combined treatment with the preparations under study produced significant positive effects on the course of the disease. The highest effect was achieved by the treatment started one hour postinfection. The local manifestations in all the treated animals were limited to the development of erythema of the mucosa in 24 hours which completely disappeared in 4 days.

When the treatment was begun later, 48 hours postinfection, the lesions were more marked. Nevertheless, the therapeutic effect was significant, and the clinical picture of local lesions in the control and experimental groups differed greatly. In the control group the mean score (the degree of intensity of clinical manifestations) was 2.8, and morphologically the foci of herpetic lesions showed a trend for confluence and were characterized by marked exudative inflammatory changes. In the experimental groups, the degree of clinical mainfestations was 2.2 and 1.8 for the animals received the gel alone and those treated by the combined method, respectively. The process showed no progress, no confluence of the foci or exudation were observed.

The maximum positive effect was recorded on the 5th day postinfection in the group of animals which received combined treatment. The foci of herpetic eruptions opened, rapid healing of erosions with epithelization of the mucosa occurred. The local positive effect prevented the development of complications. In the experimental groups the number of paralysed animals decreased to 20% and 30% whereas in the control group it was observed in 100%.

In further observations (at 7 days) the lethality in control was 80% and in the experimental groups 20% and 40%, respectively. Another experimental confirmation of the therapeutic effect of L-lysine-α-oxidase was obtained by the analysis of the contents of herpetic vesicles for HSV-2 biological activity. Titrations of the virus were done in Vero cell cultures infected with the contents genital herpetic vesicles. In the control group, an increase of biological activity of the virus was observed. In the experimental group where the treatment was started 48 hours postinfection, a decrease in the activity of the virus passaged in the cell culture by 3-4 lg TCD₅₀ was observed.

Thus, preparations of L-lysine-α-oxidase activity inhibited the development of genital herpes infection, and the combined treatment was found to be most effective.

### VII. Clinical studies of the antiherpes effect of L-lysine-α-oxidase

The clinical trials of L-lysine-α-oxidase were carried out in the Central Dermato-Venereological Institute of the USSR Ministry of Public Health. A group of 30 volunteer patients suffering from recurrent herpes affecting the skin and buccal and genital mucosa was selected. At the time of the relapse the patients were not treeted by any other medicines. In the course of the trials the duration of the relapse was found to be shorter by 2 days in 28 patients, and epithelisation of lesions was much more rapid. When the treatment was given at the very beginning of the relapse, the area of the focus of lesion was reduced significantly. The preparation in the gel form was most effective, facilitating more rapid healing of the erosive eruptions on the mucosa in genital herpes and decrease of pruritis in the focus of lesion area.

### VIII. Inhibition of HIV production by highly purified lysine oxidase

MT-4 cells (a continuous line of human T-lymphocytes propagated in vitro) sensitive to the cytopathic effect of the virus were used for investigation of the inhibiting effect of lysine oxidase on HIV virus production (HIV-1 type). The effect of the enzyme was compared to that of the standard preparation widely used at the present time for treatment of patients with AIDS - azidothymidine (AZT). To the wells of a 24-well plate under sterile conditions were added 1.8 ml of MT-4 cell suspension with a concentration of 5x100,000 cells/ml in the RPMI-1640 culture medium (100 U/ml ampicillin) containing different concentrations of the preparations.

Lysine oxidase preparations were prepared in the following way, the initial enzyme in the amount of 100 µl (specific activity 142 U/mg) was diluted with medium RPMI-1640 at a ratio 1:50 (to 5 ml); then from this solution 4.5 µl lysine oxidase were collected with a sterile pipette and added to 18 ml of cell suspension. 1.8 ml portions were added to each well and the medium or the virus-containing fluid were added to the volume of 2 ml. This made the final concentration of lysine oxidase 1:1000 U/ml of the culture medium. To the control wells 0.2 ml of the medium per well were added instead of the virus. Further decrease of the enzyme concentration to 1:10,000 - 1:1000000 were achieved by standard 10-fold dilutions.

The virus for the tests on the preparations was obtained from the culture medium of MT-4 cell cultures and pre-titrated for the cytopathic activity. The virus was added to the wells in a dose of 0.0001 - 0.00001 CPD (the cytopathic dose) in 0.2 ml and the volume in each well was adjusted to 2 ml. The antiviral effect of the enzyme was compared with that of the standard dose of AZT (3 µM/ml) .

**Table 10**

| The toxic effect of the preparations on MT-4 cells | | | | |
|---|---|---|---|---|
| Preparation | Lysine oxidase concentration in U/ml | | | |
| | 0.001 | 0.0001 | 0.00001 | 0.000001 |
| Lysine oxidase AZT | toxic | moderately toxic | not toxic | not toxic |

As will be seen from the results in Table 11, AZT in a dose of 3 µM/ml caused complete inhibition of the virus, lysine oxidase did that in doses of 0.0001-0.00001 U/ml. At concentrations of lysine oxidase lower then 0.000001 no decrease in the initial titre of HIV was observed. As will be seen in Table 10, an increase in the concentration of lysine oxidase had a cytotexic effect on the cells.

Thus, the highly purified preparations of lysine oxidase in concentrations of 0.0001-0.00001 E/ml completely blocked HIV reproduction in vitro without causing any cytotoxic effect on the cells .

**Table 11**

| The antiviral effect of lysine oxidase as compared with that of AZT or MT-4 cell line | | | | | | |
|---|---|---|---|---|---|---|
| Virus dose | Day of recording | AZT 3 mM/ml | Lysine oxidase in U/ml | | | Control |
| | | | 0.0001 | 0.00001 | 0.000001 | |
| 0.00001 | 1 | n.d^{x)} | n.d. | n.d. | 0.01 | 0.001 |
| 0.00001 | 2 | n.d. | n.d. | n.d. | 0.001 | 0.0001 |
| 0.00001 | 3 | n.d. | n.d. | n.d. | 0.001 | 0.00001 |
| 0.00001 | 4 | n.d. | n.d. | n.d. | 0.001 | 0.00001 |

| | | | | | | |
|---|---|---|---|---|---|---|
| ^{x)} n.d. - not detected | | | | | | |

The use of lysine oxidase as a HIV inhibitor demonstrated its higher specificity as compared with AZT, since for virus inhibition 1000-fold less lysine oxidase is required than AZT.

### IX. Investigation of the antimicrobial range of L-lysine α-oxidase effect

The test culture were represented by the strains from the culture museum of the Laboratory of Chemotherapy exhibiting the typical species characteristics.

The value of the minimal inhibiting concentrations was determined by the method of two-fold serial dilutions in a meat-peptone broth. The highest dilution of the preparation inhibiting the microorganism growth (titre) was established.

As follows from the results presented in the Table below, the preparation has a marked inhibiting effect for cocci. The inhibiting effect of the preparation on Staphylococci aureus (strains Nos. 209,5, 340, 0394) was observed with the dilutions thereof 1:160,000 to 1:720,000. The inhibiting effect was also observed with betahemolytie streptococci (1:640). Enterococci were found not to be very sensitive to the preparation (1:16). Some sensitivity to the preparation was observed with Sarcina (1:25000), aerobic spore-forming bacilli (1:320), and Listeria (1:640).

The range of antimicrobial effect of the preparation

| Strain | Titre |
|---|---|
| Staphylococcus aureus 209 | 1:320 000 |
| Staphylococcus aureus 5^{a} | 1:160 000 |
| Staphylococcus aureus 340 | 1:320 000 |
| Staphylococcus aureus 0394 | 1:720 000 |
| Streptococcus pyogenes 9389 | 1:640 |
| Streptococcus pyogenes 12 | 1:640 |
| Streptococcus faecalis 8 | 1:16 |
| Sarcina lutea | 1:25 |
| Bacillus subtilis | 1:320 |
| Listeria monocytogenes | 1:640 |
| Escherichia coli 60 | <1:6 |
| Proteus vulgaris 247 | <1:6 |
| Proteus mirabilis 10 | <1:6 |
| Proteus rettgeri 2 | <1:6 |
| Pseudomonas aeruginosa 387 | <1:6 |
| Providencia stuartii | <1:6 Enterobacter aerogenes 980 <1:6 |
| Klebsiella species | <1:6 |
| Klebsiella pneumonie 122 | <1:6 |
| Citrobacter species | <1:6 |
| Candida albicans | 1:400 |
| Candida utilis | 1:200 |
| Mucor | 1:3200 |

Escherichia coli, Proteus, Pseudomonas aeruginosa, Enterobater, Klebsiella sp., Citrobacter are resistant to the preparation (<1:6). The yeast-like fungi of the genus Candida are sensitive to the enzyme (1:200 - 1:400) as well as Mucor (1:3200).

Thus, the investigations established that L-lysine-α-oxidase obtained according to the claimed process is highly active against gram-positive bacteria. The highest activity of the enzyme was found against Staphylococcus aureus including the strains resistant to the antibiotics widely used in medical practice such as benzyl penicilline, tetracyclines, levomycetin, erythromycin, oleandomycin.

By its range of the antimicrobial effect L-lysine-α-oxidase is close to lysozyme which has found application in otorhinolaryngology, ophthalmology in suppurative complications after operations on the eyes, in surgical practice for treatment of wounds and burns, in gynecology for treatment of erosions, in stomatology for treatment of catarrhal and ulcerative stomatites.

The high activity of L-lysine-α-oxidase according to the invention against the antibiotic-resistant strains of Staphylococcus aureus offers the prospects for the application thereof in suppurative pathology caused by the microflora sensitive to it.

### X. Investigation of the wound-healing effect of L-lysine-α-oxidase on the model of mouse skin carcinoma induced by methylchelanthrene

The experiments were carried out in C57BL mice weighing 20-25 g. The experimental carcinomas were produced by rubbing into the animals' skin of 0.5% methylcholanthrene solution in acetone for 6 months. The development of carcinomas in the experimental and control groups was followed for 3-6 weeks.

The preparation in gel with a concentration of 70-100 µg/ml was applied to the ulcerated skin areas. The control group received a gel without L-lysine-α-oxidase. The results indicate that the L-lysine-α-oxidase preparation is significantly conductive to healing of the carcinoma wounds. The daily single rubbing of LO gel into the wounds for 3 weeks reduced the size of the carcinomas by 40%. After a longer application of the gel (6 weeks) the effect rose to 50%.

Thus, the results attest to the marked wound-healing effect of the L-lysine-α-oxidase preparation even in cases when the skin lesions are caused by the tumors induced by chemical carcinogens.

### XI. The immunomodulating activity of L-lysine-α-oxidase

The immunopotentiating activity of LO was assessed in the model of endogenous colony formation using mice of F/CBA C57 BL line according to the method described in a monograph (Petrov, Khaitov-Control and Regulation of Immune Response, 1981, p.310). The animals were inoculated twice intravenously with L-lysine-α-oxidase in a dose of 0.5 mg. On the third day the mice were irradiated with a dose of 600 R (roentgen) in an apparatus RUM-17. After irradiation the preparation was given three more times in the same dose. The animals were divided into 4 groups, 10 mice in each:
1. Irradiated, no immunization with L-lysine-α-oxidase.
2. Irradiated, immunized.
3. Irradiation + administration of lymph node cells of the parental genotype.
4. Irradiation + administration of lymph node cells of the parental genotype + administration of L-lysine-α-oxidase.

A dose of mouse lymphocytes causing 50% inactivation (colonyforming cells-CFC) was one million cells.

At 9 days the mice were sacrificed, their spleen removed, and after fixation the number of colonies on the surface of the organ was counted.

The cells of NT-4 line (human lymphoblastoid cell line cultivated in vitro) were grown in RPMI-1640 medium containing 10% embryonal calf serum and 100 U/ml ampicillin. The cells were counted in Goryaev chamber.

The rate of DNA synthesis was determined by ³H-thymidine incorporation into the acid-insoluble fraction. The final concentration of the label was 5 µCi/ml. After 1 hours of incubation of the cells with the labelled precursor, 5% trichlorcacetic acid cooled to 4°C was added to MT-4 cell sediment (1 million cells) which was washed 3 times with the cold acid and then dissolved in 1 ml of 0.1 N NaOH. To count radiactivity, 0.1 ml of the sample was put on the filters. The radioactivity of the samples was recorded on a spectrometer in a toluene scientillator (10 ml) for 1 min.

Table 12 presents the results of determinations of the immunopotentiating activity. It will be seen that administration of the preparation caused a significant stimulation of CFC (253.5%) which attested to the presence of marked immunopotentiating properties of L-lysine-α-oxidase. It may also be seen that the immunopotentiating activity of the protein persisted also in cases of inhibition of the process of endogenic formation of the colonies by cells of the parental genotype lymph nodes. The difference between the experiment and the control was 181% . Thus, the results demonstrated a high immunopotentiating activity of L-lysine-α-oxidase in vivo.

**Table 12**

| Stimulation of endogenous colonies formation by 10 | | |
|---|---|---|
| Preparation | Mean No.of endogenous colonies per spleen of sublethally irradiated mice after 10 administration | Mean No.of endogenous cologies per spleen of sublethally irradiated mice after administration of 1 min of parental genotype lymphocytes and LO |
| Control (physiological solution) | 12.18 + 2.76 | 3.8 + 0.53 > 0.05 |
| LO preparation | 30.9 + 4.6 | 6.9 + 1.22 |

The stimulation by L-lysine-α-oxidase of replicative synthesis in vitro of MT-4 lymphoblastoid cells was analysed. The results of the study of the rate of DNA synthesis by incorporation of labelled thymidine at different stages of growth demonstrated a significant difference in the rate of DNA synthesis in experimental and control cultures at 3 days. By the 7th day of growth in the presence of L-lysine-α-oxidase the intensity of DNA biosynthesis exceeded the control level by more than 2-fold (224%). L-lysine-α-oxidase concentration in the culture fluid of MT-4 cells was 10 times lower than that exerting the cytotoxic effect on the cells. Consequently, the high immunopotentiating activity of L-lysine-α-oxidase has been confirmed by the experiments in vitro.

The invention is illustrated by, but not limited to, the following examples.

### Example 1

A culture of Trichoderma harzianum Rifai F-180 grown in wortagar for 14 days in a thermostate at 28°C was transferred into the medium with wheat bran having the following composition: wheat bran 10 g, NaNO₃ 0.85 g, H₂O 10 ml, and grown under similar conditions for 14 days. 0.5 g of the inoculum (the fungus with wheat bran) was placed into a 250 ml capacity flask containing a medium of the following composition: wheat bran 4 g, NaNO₃ 0.7 g, water 100 ml. The initial pH was 5.5. The producer was cultivated under aeration conditions on a shaker type 357 (Poland) at 28°C, 130 rpm with the amplitude No.6, for 5 days. The resulting culture fluid was used for isolation of the enzyme.

The activity of L-lysine-α-oxidase was determined spectrophotometrically by the amount of H₂O₂ formed in the course of the enzymatic reaction. The unit of the enzyme activity was considered to be the amount of the enzyme catalysing formation of 1 nmol H₂O₂ in one minute under the standard conditions per 1 ml of liquid or 1 mg of protein. The enzyme activity was 0.8 U/mg of protein. The homogeneous preparation was obtained by the above-described method.

### Example 2

A culture of Trichoderma harzianum Rifal F-180 grown in wortagar for 14 days in a thermostate at 28°C was transferred into the medium with wheat bran having the following composition: wheat bran 10 g, NaNO₃ 0.85 g, H₂O 10 ml, and grown under similar conditions for 14 days. 0.5 g of the inoculum (the fungus with wheat bran) was placed in a 250-ml capacity flask containing a medium of the following composition: wheat bran 6 g, NaNO₃ 1.5 g, water up to 100 ml, the initial pH was 6. The producer was cultivated for 5 days. The resulting culture fluid was used for isolation of the enzyme.

The activity of L-lysine-α-oxidase was determined as described in Example 1. The activity was 0.6 U/mg of protein. The homogeneous preparation was obtained by the above-described method.

### Example 3

The seed material of Trichoderma harzianum Rifai F-180 was grown as described in Example 1. The fermentation medium had the following composition: wheat bran 5 g, NaNO₃ 0.9 g, water up to 100 ml. The initial pH of the medium was 5.8. The producer was cultivated for 5 days under aeration conditions at 120 rpm at an amplitude No.6. The activity of L-lysine-α-oxidase of the fungus grown under these conditions was 1.2 U/mg of protein. The homogeneous preparation was obtained by the above-described method.

### Example 4

The seed material of Trichoderma harzianum Rifai F-180 was obtained as described in Example 1; further the producer was grown by submerged cultivation in a medium; wheat bran 10 g, NaNO₃ 0.79 g, water 10 ml. The activity of L-lysine-α-oxidase obtained by different cultivation methods is shown in Table 13.

**Table 13**

| Method | Day of growth | Activity, U/mg |
|---|---|---|
| Surface cultivation (known) | 5 | 0.3 |
| | 8 | 0.6 |
| Submerged cultivation | 5 | 1.2 |
| | 8 | 0.8 |

It will be seen from the Table that the submerged cultivation the maximum of the enzyme formation is observed at 5 days of growth whereas in the surface cultivation at 8 days of growth.

In submerged cultivation, however, of great importance is the medium used for the growth of the seed material, the mandatory component of which is wheat bran.

The influence of the medium for the seed material on the activity of the enzyme grown by different methods is shown in Table 14.

**Table 14**

| Method | Capek medium, wort-agar medium | Wheat bran medium |
|---|---|---|
| Surface cultivation(known) | +A | + |
| Submerged cultivation | - | + |
| Note: +A - production of L-lysine-α-oxidase - no L-lysine-α-oxidase. | | |

The technology of preparation of the initial product for isolation and purification of L-amino acids oxidases by the submerged cultivation has one more advantage: the spread of the fungus is limited (flask, fermenter). In the surface cultivation the fungus infects not only the equipment and people but also the environment.

## Claims

1. The strain Trichoderma harzianum Rifai, a producer of L-lysine-α-oxidase deposited on 12.01.82 at the All-Union collection of industrial microorganisms of the All-Union Research Institute of Genetics and Selection of industrial microorganisms, the registration number F-180.

2. A process for preparation of L-lysine-α-oxidase comprising cultivation of Trichoderma sp. in a fermentation medium containing wheat bran as the source of carbon, a source of inorganic nitrogen, and water, subsequent cultivation under aeration conditions to the maximum accumulation of the target product characterized in that Trichoderma harzianum Rifai F-180 strain is cultivated by the submerged method and the fermentation medium components are used at the following ratio (% by weight): wheat bran 4-6, the source of inorganic nitrogen 0.7-0.9, water - the rest, the initial pH of the medium is 5.5-6, and the cultivation is carried out under aeration conditions of 100-130 rpm.

3. The application of L-lysine-α-oxidase produced by the strain according to claim 1 as an inhibitor of viral and bacterial activity.

4. The application of L-lysine-α-oxidase produced by the strain according to claim 1 as an immunopotentiator.

5. The application of L-lysine-α-oxidase produced by the strain according to claim 1 as a stimulator of healing of skin lesions.
